Europäisches Patentamt

**European Patent Office**

Office européen des brevets

(11) Publication number : **0 318 561 B1**

# EUROPEAN PATENT SPECIFICATION

(45) Date of publication of patent specification :
**13.12.95 Bulletin 95/50**

(21) Application number : **88905488.8**

(22) Date of filing : **17.05.88**

(86) International application number :
**PCT/US88/01651**

(87) International publication number :
**WO 88/09950 15.12.88 Gazette 88/27**

(51) Int. Cl.⁶ : **G02C 7/04,** G02C 7/06,
G02B 1/00

## (54) MULTIFOCAL OPHTHALMIC LENS

(30) Priority : **01.06.87 US 56050**

(43) Date of publication of application :
**07.06.89 Bulletin 89/23**

(45) Publication of the grant of the patent :
**13.12.95 Bulletin 95/50**

(84) Designated Contracting States :
**AT BE CH DE FR GB IT LI LU NL SE**

(56) References cited :
**DE-A- 2 702 117**
**FR-A- 1 319 800**
**US-A- 3 431 327**
**US-A- 3 542 461**

(56) References cited :
**US-A- 4 055 378**
**US-A- 4 073 579**
**US-A- 4 162 122**
**US-A- 4 338 005**

(73) Proprietor : **PORTNEY, Valdemar**
**7 Alassio**
**Irvine, CA 92720 (US)**

(72) Inventor : **PORTNEY, Valdemar**
**7 Alassio**
**Irvine, CA 92720 (US)**

(74) Representative : **Allden, Thomas Stanley et al**
**A.A. THORNTON & CO.**
**Northumberland House**
**303-306 High Holborn**
**London WC1V 7LE (GB)**

## Description

This invention relates to ophthalmic lenses, e.g., intro-ocular lenses (IOLs), contact lenses, and corneal implant and onlay lenses; and it concerns the problem of providing ophthalmic lenses which successfully handle bifocal, and other multifocal, corrections.

Where spectacles, or eyeglasses, are used, the movement of the eyes relative to the lenses selects the different focal powers for near and far vision. Where ophthalmic lenses are used, other means must be provided for such selection. At least three types of lens designs, primarily for contact lenses, have been suggested as possible means of satisfying this need. In each of these types of contact lens designs, problems have been encountered, primarily due (a) to the need for centering of the lens on the eye, and (b) to the effects of normal changes in the size of the eye's pupil.

One form of multifocal ophthalmic lens design is illustrated by US-A-4593981 which discloses a bifocal contact lens designed to correct for near vision in the centre portion of the lens and for far vision in the peripheral portion of the lens. With this type of lens, centring on the eye is essential for satisfactory performance; and correct size of the optical zones is also important. If either of these requirements is not met, a lens of this type can produce diplopia or fringing.

Another form of multifocal ophthalmic lens design is illustrated in US-A-4580882 which discloses a multifocal contact lens having optical power which continuously varies from minimum at the optical centre point to maximum at the periphery of the optical zone. Usually this progressive (aspheric, variable focus) type of lens is constructed with a centrally placed small zone of constant curvature from which aspheric curves are grown towards the periphery in all meridians. The central area serves as the power for the distant correction, while the peripheral curves provide a varying amount of additive plus power for the near point. The curves may be placed on the front surface in which case they increase in convexity, or on the back surface in which case they decrease in concavity (flatten). If the surface of progressive curvature is placed on the front of the lens, the tear layer interferes with the lens performance. If the progressive curvature is placed on the back surface of the lens, this will affect the fitting characteristics of a contact lens. In both cases the image is "undercorrected", which is more natural for human vision. This "progressive" power lens has the advantage that flare or diplopia does not occur if the lens is slightly off-centre. However, pupil size affects vision with this lens, as it does with the lens discussed in the preceding paragraph.

A third form of multifocal ophthalmic lens design is illustrated in US-A-4549794 and US-A-4573775 which disclose bifocal contact lenses of the segmented type, i.e., lenses in which a segment having different refractive characteristics is embedded at a selected position in the lens body. The segments are positioned along the vertical axes. Lenses of this type do not have symmetry around their centres; and they require some form of ballast to assure maintaining the desired orientation. Deviation from proper orientation affects the image quality.

One attempt to solve the centralization and orientation problems in a bifocal lens is represented by US-A-4162122 which discloses a zonal bifocal contact lens in which annular concentric zones alternate between the near and far vision powers. This is accomplished by providing an anterior lens surface having characteristics similar to a Fresnel lens, except that sharp zonal edges are avoided. This structure has disadvantages due to the multiple diffraction caused by the abrupt curvature change of the lens surface from one zone to another; and also due to uncertainty as to the tear layer distribution on the anterior surface of the contact lens.

Designs similar to those described above are proposed also for intraocular lenses: e.g., US-A-4636211 and EP-A-0140063. Both of them describe several zones of different curvatures for far and near vision. Continuity of the surface curvature is also important for an intraocular lens because it has an effective optical zone of only 3 mm diameter for daytime vision. Disruption of this relatively small optical zone can reduce the image performance. Besides, such lenses suffer from all of the problems described for contact lenses.

Multifocal ophthalmic lenses previously developed have, in general, tended to provide unstable optical systems because of random changes in lens position relative to the pupil of the eye, and also because of changes in the pupil size which significantly affect the imaging performance.

In accordance with the present invention as claimed there is provided an ophthalmic lens which is adapted to be carried by the eye for providing variable vision correction power, said lens comprising at least one annular zone, the corrective power varying radially outwardly in said annular zone from a first vision correction value to a second vision correction value and then to a third vision correction value which is between the first and second vision correction values and at least a portion of the variation of the corrective power in said annular zone being continuous and progressive.

Preferably, the invention combines (a) a series of alternating power zones with (b) a continuously varying power within each zone, as well as in transition from one zone to another. In other words, a plurality of concentric zones (at least two) are provided in which the variation from far to near vision correction is continuous, i.e., from near correction focal power to far correction focal power, then back to near, and again back to far, or vice versa. This change is continuous (progressive), without any abrupt correction changes, or "edges". The construction may also be such that the radial width of the zone for far-to-near transition is larger than the radial width of the zone for near-to-far transition, in order to provide "undercorrected" performance of the lens overall.

Two versions of the invention will be disclosed hereinafter. In the first version, continuous, alternating power variation is accomplished by a continuously changing curvature of the lens posterior surface, thereby altering the angle of impact of light rays on the eye.

The second version accomplishes continuous, alternating power variation by creating non-homogeneous surface characteristics having refractive material indexes which continuously vary in the lens radial direction (out from the optical axis). This technique has a similar effect to the aspherizing of the surface by utilizing continuous curvature variation as described above. Such surface refractive variations may be provided either on the convex anterior or on the concave posterior surface of the lens. This variation in the refractive index may be accomplished by ion-implantation techniques. This approach is particularly suitable for a corneal implant (corneal inlay) or a corneal onlay (the former is implanted inside the cornea, and the latter is placed between the corneal epithelium layer and the anterior surface of the cornea.

In order that the invention may be well understood there will now be described some embodiments thereof, given by way of example, reference being made to the accompanying drawings, in which:

Figures 1-3 are sketches illustrating the three general prior art approaches discussed above;

Figure 4 is an enlarged cross-section of a contact lens emboding the invention, having a multi-zone, continuously-varying posterior surface (the exaggerated dimensions of the varying lens surface illustrate concepts of the present invention);

Figure 5 is a front view of the posterior of the lens of Figure 4, illustrating the concentric arrangement of the peaks and valleys of the lens surface;

Figures 6A and 6B are further enlarged closeups of a small portion of the posterior lens surface of Figure 4, used to explain the focusing effects of light rays from near and far locations;

Figure 7 is a cross-section of another contact lens embodying the invention, in which the forward, or anterior, convex surface of the lens has a varying refractive index gradient of the material in the radial direction, in order to obtain the same result as the lens in Figures 4, 6A and 6B, but using refractive index variations instead of surface curvature variations; and

Figures 8A, 8B and 8C are cross-sections, respectively, of a corneal implant lens, a corneal onlay lens and an intra-ocular lens, anyone of which may have its anterior or posterior surface formed similarly to the posterior contact lens surface shown in Figure 4.

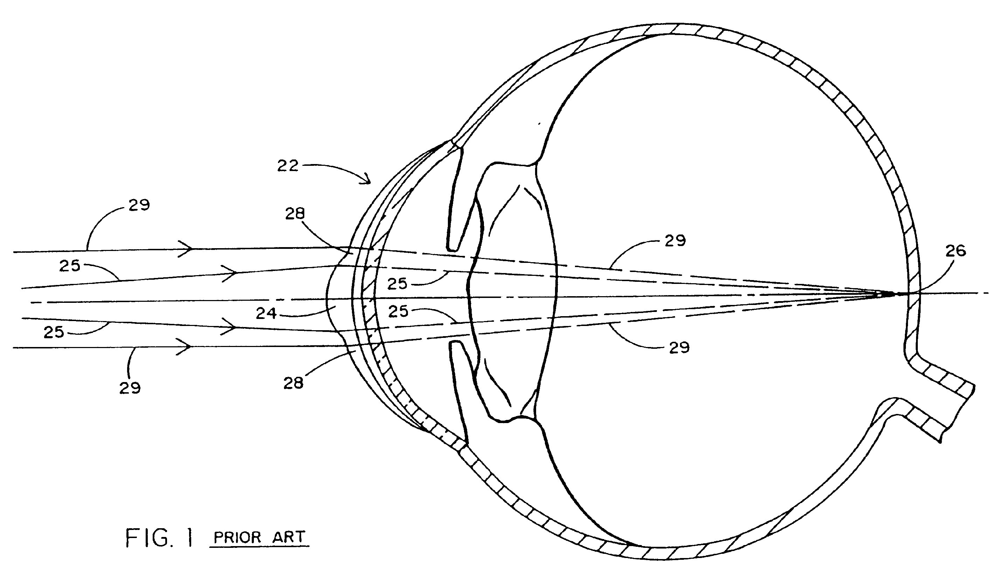

Referring first to Figure 1, there is illustrated a prior art effort to solve the problem of forming bifocal contact lenses. A contact lens 22, shown in cross-section, has a central portion 24 designed to focus light from near objects on the retina 26, as shown by the inner lines 25 representing light rays. Contact lens 22 has a peripheral portion 28, which is designed to focus light from far objects on the retina 26, as shown by the outer lines 29 representing light rays.

The bifocal lens of Figure 1 can only focus objects located at specific distances (far and near) and separated. Also, it clearly is subject to problems due to any displacement from centrality of the lens on the eye, and due to changes in the size of the eye's pupil.

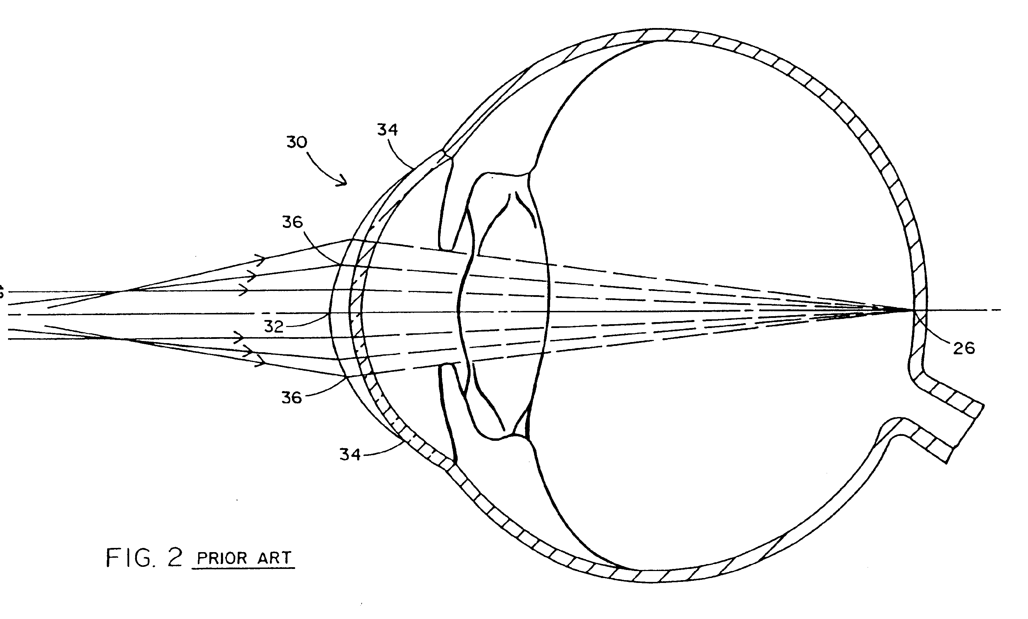

Figure 2 illustrates another prior art effort to solve the problem of forming varying focus contact lenses. A lens 30 is shown is cross-section, having its centre at 32 and its periphery at 34. Because of its continuously changing curvature from its centre to its periphery, it provides a continuous change of focusing power from viewing far objects at the centre to viewing near objects at the periphery, within a range of, say, two diopters, as illustrated by the lines 36 representing retina-focused light rays. The central ray at 32 is from the farthest object viewed, and the focused rays 36 are progressively from nearer objects, as their contact points on lens 30 move closer to its periphery.

This continuous variation in lens power has advantages over the arrangement of Figure 1, from the standpoint of being more easily accepted by the retina and the brain. It also is less susceptible to centring problems, i.e., flare or double image has not been reported for this type of lens it is slightly decentred. However, it is negatively affected by variations in pupil size and by large decentration; and also this lens type tends to create fitting problems between the posterior surface of the lens and the cornea.

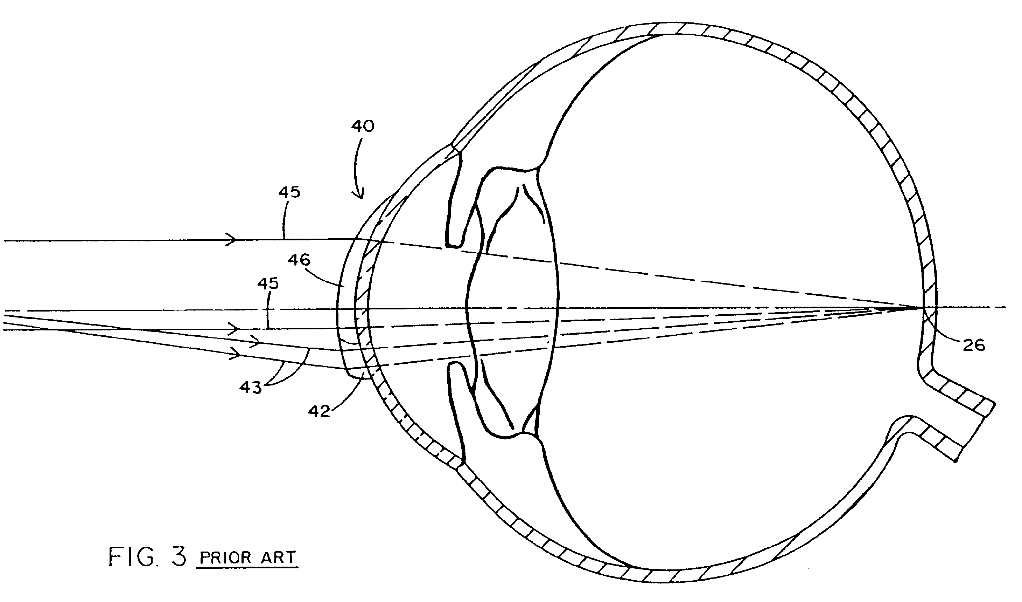

Figure 3 illustrates a third prior art approach, which is conceptually similar to bifocal spectacles. A contact lens 40 includes an embedded segment 42 formed of material having a different refractive index from the remainder of the lens. The segment 42 is used to provide near vision correction, as shown by rays 43. The remaining rays 45, which come from farther objects, are focused at the retina because of the gradual change in thickness of the body 46 of the lens.

The lens of Figure 3 does not have central symmetry, and requires the use of ballast to maintain the desired orientation. Deviation from proper orientation affects the image quality.

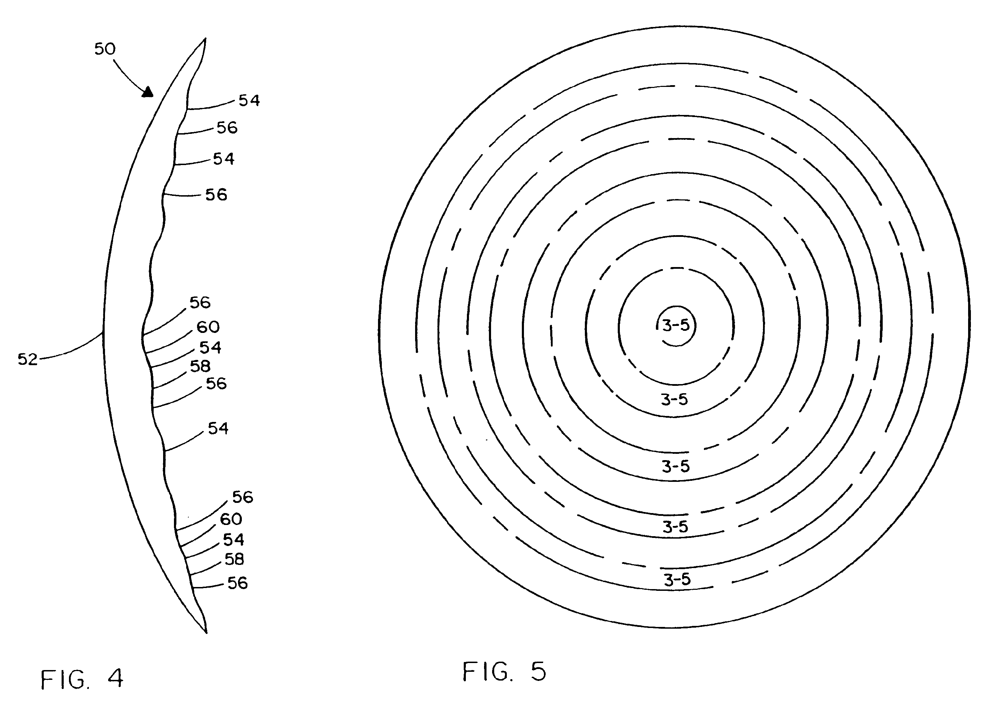

In order to minimize the problems due to the need for centring, due to pupil size variation, and due to fitting requirements (progressive type), the embodiment of the present invention shown in Figure 4 uses several zones, each of which includes at least two progressive power changes between near correction and far correction. In other words, in a three zone contact lens of the type shown in Figure 4, the progressive variation of the Figure 2 lens would be repeated six times, three times as a variation from lower to higher power, and three times as a variation from higher to lower power. The lens is constructed with a small centrally placed zone of constant curvature to form the power for middle correction. From it the curvature changes to far correction, then to near correction, passing through the middle correction. This alternation is continued to form several zones. It is considered desirable, but not necessary, to have slower variation from far-to-near correction and faster variation from near-to-far correction, in order to form in general some "undercorrection", as found in most visual instruments.

Figure 4 shows a contact lens 50, the outer convex surface 52 of which has a smooth arcuate shape, and the inner surface of which has an undulating surface, as seen in very exaggerated dimensions in the figure. The inner surface of a contact lens is much preferred as the variable power, or undulating, surface, because the space between the lens and the eye will be filled with tears having a predetermined varying depth. This predetermined depth of tears permits the refractive effect of the tears to be compensated for in the design of the lens. If the undulating surface were formed on the exterior surface 52 of the lens, the uncertainty of the tear layer distribution would tend to prevent optimal imaging.

The view from the right side (posterior) of the proposed lens is shown in Figure 5. The zones shown by dashed lines in reality have a continuous curvature, e.g., the numbers inside each zone represent the range of diopters.

The lens has a constraint placed on its posterior surface determined by the fitting characteristics for a given cornea. Usually, the back surface of a contact lens (base curve) is from 0.5 to 1.0 diopter steeper than the corneal shape, which translates to approximately 0.1 mm to 0.2 mm distance between the inner, or posterior, surface of the contact lens and the front surface of the cornea. This is usually the case in single focus contact lenses. The undulations in the posterior surface of lens 50 in Figure 4 represent a distance difference, or maximum depth, of less than 20 microns between the peaks 54 and valleys 56 (as they would appear if Figure 4 were rotated 90° counterclockwise). The zones are placed within a dimensional range (diameter) of the lens of about 5 mm; and within this range the peak-to-valley differences are always much smaller than the available gap between lens and cornea. This significantly simplifies the fitting requirements, as the regular single vision lens can be fitted first and then replaced with the multifocal lens of similar base curve configuration.

The peaks 54 and valleys 56, which occur at points tangent to circles having their centres at the centre of the outer curvature 52 of the lens, represent the intermediate focal distances, or optical correction powers, of the continuously varying correction values of the lens 50. The higher and lower corrective powers in each zone of the four concentric zones shown in Figure 4, occur as the undulating curve progresses from one peak 54 to the adjacent valley 56, and then back to the next peak 54. A zone is considered to include a complete cycle, i.e., from the intermediate power through the high power, then back through the intermediate power to low power, and finally back to the intermediate power.

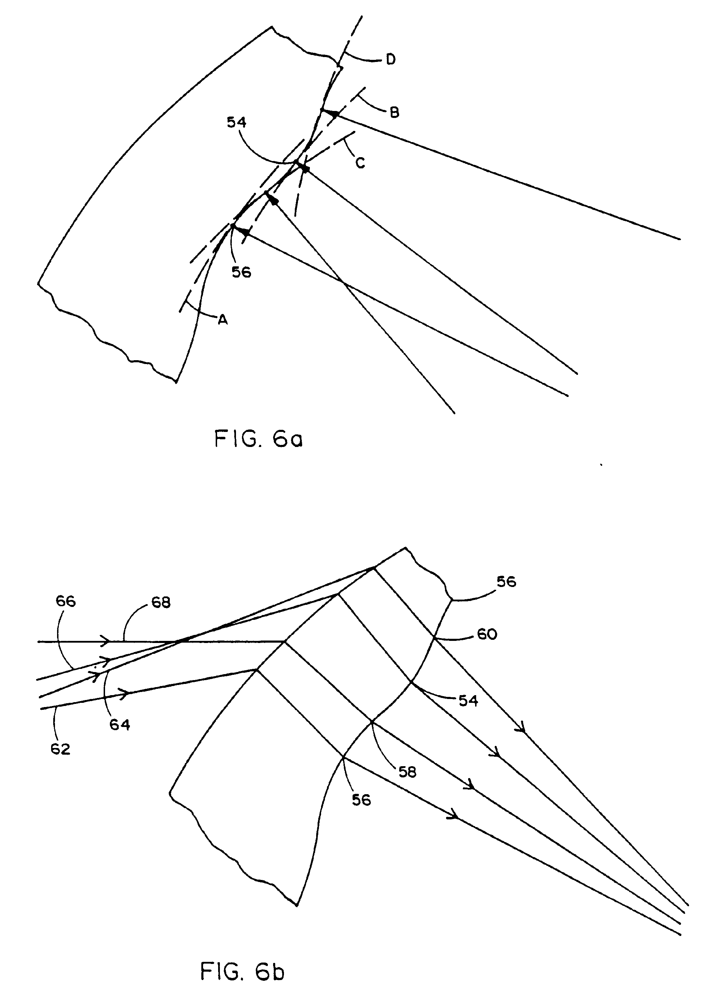

This is illustrated in the highly enlarged Figures 6A and 6B. As seen in Figure 6B, a first ray 62, from an object at an intermediate distance, passes through a valley 56 in the posterior lens surface. A second ray 68, from an object at a far position (lower power correction), passes through a portion 58 of the undulating curve formed as that curve progresses toward the adjacent peak 54 in the posterior lens surface. A third ray 66, from an object at an intermediate distance, passes through the peak 54. A fourth ray 64, from an object at a near position (higher power correction), passes through a portion 60 of the undulating curve formed as that curve progresses toward the next valley 56 in the posterior lens surface. All of the rays 62, 64, 66 and 68, are focused at the retina, as shown.

Figure 6A illustrates the principles used in formation of the undulating curve, by considering the curve as comprising progressive focal powers corresponding to individual lenses having curvatures as illustrated by the dashed lines A, B, C and D. The arcuate line A is tangent to the valley 56. The arcuate line B is tangent to the peak 54. The arcuate line C is tangent to the undulating curve at a point midway between the valley 56 and the peak 54. And the arcuate line D is tangent to the undulating curve at a point midway between the peak 54 and the next valley. For full progressivity, each point on the undulating curve has a different radial centre from the centres of the adjacent points.

The undulating surface of the lens is preferably formed by a computer-controlled machining apparatus. The computer program of this computer numerically controlled (CNC) machine generates a large number of closely spaced points (coordinates), which represent the multifocal surface for the given design requirements (curvature, range of accommodation, number of zones, etc). The computer of the CNC machine then forms a linear approximation between these points to generate a surface with good approximation to the ideal surface contour. A sample of the points is provided by the following table. In the fabrication of a three zone contact lens, with the three zones being identified by the code numbers, "13M3", "23M3", and 33M3", respectively, an exemplary set of coordinates is set forth in the table. The first column of figures represents the radius of the lens aperture (distance from the lens centre). The third column represents the surface coordinates along the optical axis. And the second column represents the deviation of the above surface from the spherical surface of the mid-range power.

## 13M3

| | | |
|---|---|---|
| .417 | −.0001 | .012 |
| .589 | −.0006 | .0236 |
| .722 | −.0013 | .035 |
| .833 | −.002 | .0465 |
| .932 | −.0023 | .0584 |
| 1.021 | −.0024 | .0705 |
| 1.102 | −.0022 | .0829 |
| 1.179 | −.0018 | .0956 |
| 1.25 | −.001 | .1086 |
| 1.318 | −.0004 | .1215 |
| 1.382 | 0.00 | .1343 |
| 1.443 | .0001 | .1457 |

## 23M3

| | | |
|---|---|---|
| 1.443 | .0001 | .1467 |
| 1.616 | −.0003 | .1839 |
| 1.687 | −.001 | .2001 |
| 1.742 | −.0018 | .2127 |
| 1.789 | −.0025 | .2238 |
| 1.829 | −.0029 | .234 |
| 1.866 | −.003 | .2437 |
| 1.9 | −.0029 | .253 |
| 1.932 | −.0026 | .262 |
| 1.961 | −.0021 | .2709 |
| 1.989 | −.0017 | .2793 |
| 2.016 | −.0015 | .2872 |
| 2.041 | −.0015 | .2948 |

## 33M3

| | | |
|---|---|---|
| 2.041 | −.0015 | .2948 |
| 2.174 | −.0021 | .3346 |
| 2.229 | −.0029 | .3517 |
| 2.271 | −.0038 | .3646 |
| 2.306 | −.0046 | .3758 |
| 2.337 | −.005 | .3861 |
| 2.366 | −.0051 | .3957 |
| 2.392 | −.005 | .4049 |
| 2.416 | −.0048 | .4138 |
| 2.439 | −.0043 | .4224 |
| 2.46 | −.004 | .4306 |
| 2.48 | −.0038 | .4383 |
| 2.5 | −.0038 | .4455 |

It is understood that alternative methods of manufacture are available, such as laser ablation or moulding.

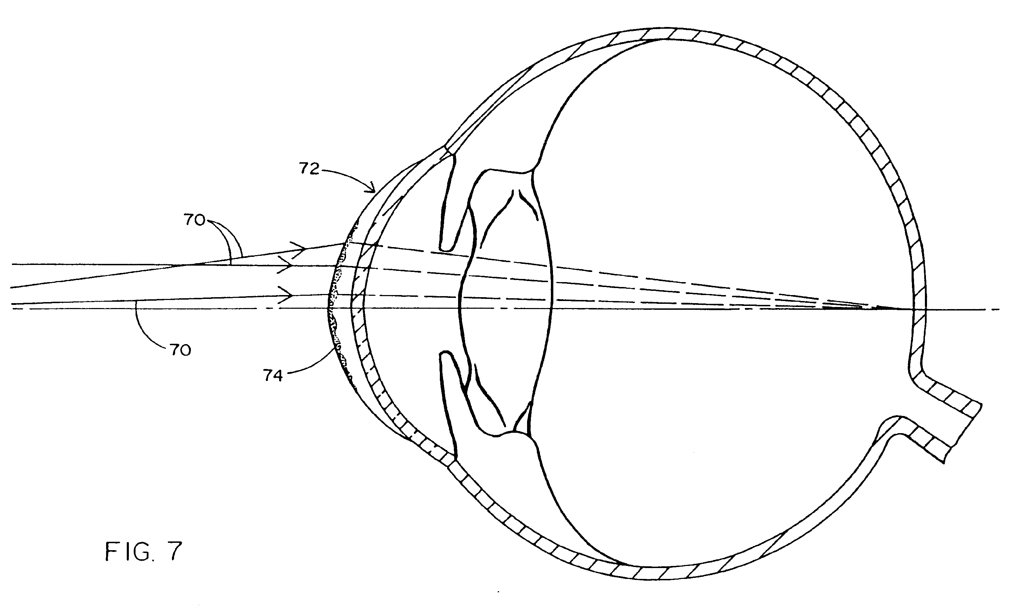

Figure 7 illustrates another embodiment of the present invention. In this figure the optical gradient is produced by variations in the refractive index of the material reached by the rays 70. The refractive variations are preferably provided on the anterior convex surface of the lens 72. This is preferred because the probable manufacturing method will utilize ion-implantation techniques to produce density variations at the surface of the lens; and it is considered a safety precaution to have the ion-implanted surface on the side of the lens away from the eye.

Although Figure 7 shows depth variations (in the shaded area 74) to illustrate the invention, in practice the refractive variations might involve density variations, rather than depth variations, or both.

In producing the lens 72 of Figure 7, ion implantation can be used to increase the index of refraction, as the result of lattice disorder produced by non-substitutional implanted ions. For example, by implantation of fused quartz thick-film-deposited on the lens substrate with ions of nitrogen or other elements, a layer of increased index of refraction is produced. The index of refraction is produced. The index refraction is directly proportional to the ion concentration per $cm^3$. The depth of penetration of the implanted ions depends on their mass and energy. Knowing the penetration characteristics and the implanted ion dose per $cm^2$, which can be determined very accurately by measuring beam current density of the system and implant time, one can calculate the profile of implanted ion concentration at varying depths. As with any particles with charges, the electromagnetic lenses and beam scanner can be used to form practically any variation of ion concentration at the substrate, and particularly to form progressive zonal lens having the optical characteristics of Figures 6A and 6B. Similar results can be achieved by using masks of varied density. The vision corrective effect would correspond to that produced by the posterior surface undulations in the lens of Figure 4.

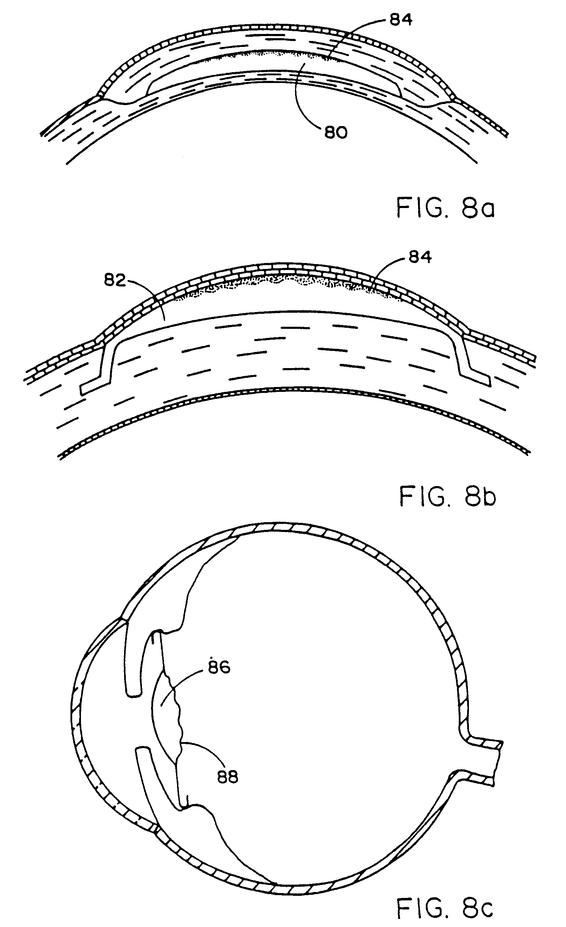

Figures 8A, 8B and 8C show, respectively, a corneal inlay lens, a corneal onlay lens, and an intraocular lens, each incorporating the concepts of the present invention. In the corneal inlay lens 80 of Figure 8A, and in the corneal onlay lens 82 of Figure 8B, the illustrated progressive zonal variations are accomplished with the variable refractive index of lens material 84, as described in conjunction with Figure 7.

In the intraocular lens 86 of Figure 8C, the posterior surface 88 is shown as an undulating surface having progressive zonal variations comparable to those in Figure 4.

Any of the three lens implants of Figures 8A, 8B or 8C could use either the surface variations or the refractive index variations, and also could use either the anterior or posterior surface as the multifocal surface.

The implanted lenses of Figures 8A, 8B and 8C are subject to the same problems as are the contact lenses, e.g., pupil size variations and decentration problems. The pupil size problems are essentially the same. The decentration problems are less pronounced with implanted lenses, but are nevertheless significant because operational procedures do not ensure centration, and, in the case of intraocular lenses, postoperative movement can be quite noticeable.

From the foregoing description, it will be apparent that the apparatus and method disclosed in this application will provide the significant functional benefits summarized in the introductory portion of the specification.

## Claims

1. An ophthalmic lens (50,72,80,82,86) which is adapted to be carried by the eye for providing variable vision correction power, said lens comprising at least one annular zone, the corrective power varying radially outwardly in said annular zone from a first vision correction value to a second vision correction value and then to a third vision correction value which is between the first and second vision correction values and at least a portion of the variation of the corrective power in said annular zone being continuous and progressive.

2. An ophthalmic lens (50,72,80,82,86) as claimed in claim 1, wherein the corrective power varies radially outwardly from the second vision correction value back to the first vision correction value.

3. An ophthalmic lens (50,72,80,82,86) as claimed in claim 1 or claim 2, wherein at least a portion of the variation in corrective power in said annular zone between the first and second vision correction values and at least a portion of the variation in corrective power in said annular zone between the second and third vision correction values are continuous and progressive.

4. An ophthalmic lens (50,72,80,82,86) as claimed in any of claims 1 to 3, wherein the corrective power in said annular zone varies continuously and progressively in a radial direction substantially completely

across said zone.

5. An ophthalmic lens (50,72,80,82,86) as claimed in any of claims 1 to 4, wherein the lens has a central zone which provides vision correction and said annular zone surrounds the central zone.

6. An ophthalmic lens (50,72,80,82,86) as claimed in claim 5, wherein the corrective power of the central zone includes a vision correction value which is between the first and second vision correction values.

7. An ophthalmic lens (50,72,80,82,86) as claimed in any of claims 1 to 6, wherein the lens includes a second annular zone surrounding the first annular zone, said second annular zone has in radially outwardly extending order a fourth vision correction value, progressive vision correction values, a fifth vision correction value and progressive vision correction values, said fourth and fifth vision correction values being different and the progressive vision correction values of the second annular zone including vision correction values between the fourth and fifth vision correction values.

8. An ophthalmic lens (50,72,80,82,86) as claimed in claim 7, wherein the first and fourth vision correction values are less than the second and fifth vision correction values, respectively.

9. An ophthalmic lens (50,72,80,82,86) as claimed in any of the preceding claims, wherein said first vision correction value is a far vision correction power and said second vision correction value is a near vision correction power.

10. An ophthalmic lens (50,72,80,82,86) as claimed in any of the preceding claims, wherein the ophthalmic lens is an intraocular lens (86).

11. An ophthalmic lens (50,72,80,82,86) as claimed in any of claims 1 to 9, wherein the ophthalmic lens (80,82) is adapted for affixation to the cornea.

12. An ophthalmic lens (50,72,80,82,86) as claimed in any of claims 1 to 9, wherein the ophthalmic lens is a contact lens (50).

13. An ophthalmic lens (50,72,80,82,86) as claimed in any of the preceding claims, wherein the lens (72) has a progressively varying index of refraction which provides the continuous and progressive variation of the corrective power.

14. A method of forming an ophthalmic lens (72) which comprises:
    preparing a lens having anterior and posterior surfaces shaped as segments of spheres which converge at the periphery of the lens; and
    causing ion-implantation on at least one surface of the lens in such a way as to cause that surface to have varying optical refractive index values, which provide a plurality of concentric optical zones, each of which zones has a higher power corrective portion and a lower power corrective portion interconnected by progressively varying intermediate portions.

## Patentansprüche

1. Ophthalmische Linse (50; 72; 80; 82; 86), die zum Tragen durch das Auge vorgesehen ist, mit veränderlicher Korrektur-Brennstärke für die Sehkraft, wobei die Linse mindestens eine ringförmige Zone umfaßt und die Korrektur-Brennstärke sich in der ringförmigen Zone in radialer Richtung nach außen von einem ersten Korrekturwert für die Sehkraft zu einem zweiten Korrekturwert für die Sehkraft und dann zu einem dritten Korrekturwert für die Sehkraft verändert, der zwischen dem ersten und dem zweiten Korrekturwert für die Sehkraft liegt, und wobei ferner mindestens ein Teil der Veränderung der Korrektur-Brennstärke in der ringförmigen Zone stetig fortschreitend ist.

2. Ophthalmische Linse (50; 72; 80; 82; 86) nach Anspruch 1, bei der die Korrektur-Brennstärke sich in radialer Richtung nach außen von dem zweiten Korrekturwert für die Sehkraft zurück zu dem ersten Korrekturwert für die Sehkraft verändert.

3. Ophthalmische Linse (50; 72; 80; 82; 86) nach Anspruch 1 oder Anspruch 2, bei der mindestens ein Teil der Veränderung der Korrektur-Brennstärke in der ringförmigen Zone zwischen dem ersten und dem zweiten Korrekturwert für die Sehkraft und mindestens ein Teil der Veränderung der Korrektur-Brennstärke in der ringförmigen Zone zwischen dem zweiten und dem dritten Korrekturwert für die Sehkraft stetig fortschreitend ist.

4. Ophthalmische Linse (50; 72; 80; 82; 86) nach einem der Ansprüche 1 bis 3, bei der die Korrektur-Brennstärke in der ringförmigen Zone sich stetig fortschreitend in einer radialen Richtung im wesentlichen über die gesamte Zone verändert.

5. Ophthalmische Linse (50; 72; 80; 82; 86) nach einem der Ansprüche 1 bis 4, bei der die Linse eine zentrale Zone aufweist, die eine Korrektur der Sehkraft bewirkt, und wobei die ringförmige Zone die zentrale Zone umgibt.

6. Ophthalmische Linse (50; 72; 80; 82; 86) nach Anspruch 5, bei der die Korrektur-Brennstärke der zentralen Zone einen Korrekturwert für die Sehkraft einschließt, der zwischen dem ersten und dem zweiten Korrekturwert für die Sehkraft liegt.

7. Ophthalmische Linse (50; 72; 80; 82; 86) nach einem der Ansprüche 1 bis 6, bei der die Linse eine zweite ringförmige Zone umfaßt, die die erste ringförmige Zone umschließt, wobei die zweite ringförmige Zone in einer radial nach außen verlaufenden Reihenfolge einen vierten Korrekturwert für die Sehkraft, fortschreitende Korrekturwerte für die Sehkraft, einen fünften Korrekturwert für die Sehkraft und fortschreitende Korrekturwerte für die Sehkraft aufweist, wobei der vierte und der fünfte Korrekturwert für die Sehkraft unterschiedlich sind und die fortschreitenden Korrekturwerte für die Sehkraft der zweiten ringförmigen Zone Korrekturwerte für die Sehkraft einschließen, die zwischen dem vierten und dem fünften Korrekturwert für die Sehkraft liegen.

8. Ophthalmische Linse (50; 72; 80; 82; 86) nach Anspruch 7, bei der der erste und der vierte Korrekturwert für die Sehkraft kleiner sind als der zweite bzw. der fünfte Korrekturwert für die Sehkraft.

9. Ophthalmische Linse (50; 72; 80; 82; 86) nach einem der vorhergehenden Ansprüche, bei der der erste Korrekturwert für die Sehkraft eine Korrektur-Brennstärke für das FernSehen aufweist und der zweite Korrekturwert für die Sehkraft eine Korrektur-Brennstärke für das Nah-Sehen aufweist.

10. Ophthalmische Linse (50; 72; 80; 82; 86) nach einem der vorhergehenden Ansprüche, bei der die ophthalmische Linse eine Linse im Augeninneren (86) ist.

11. Ophthalmische Linse (50; 72; 80; 82; 86) nach einem der Ansprüche 1 bis 9, bei der die ophthalmische Linse (80, 82) zur Anbringung an der Hornhaut vorgesehen ist.

12. Ophthalmische Linse (50; 72; 80; 82; 86) nach einem der Ansprüche 1 bis 9, bei der die ophthalmische Linse eine Kontaktlinse (50) ist.

13. Ophthalmische Linse (50; 72; 80; 82; 86) nach einem der vorhergehenden Ansprüche, bei der die Linse (72) einen sich fortschreitend verändernden Refraktionsindex aufweist, der die stetig fortschreitende Veränderung der Korrektur-Brennstärke bewirkt.

14. Verfahren zum Ausbilden einer ophthalmischen Linse (72), das umfaßt:
Vorbereiten einer Linse mit einer vorderseitigen und einer rückseitigen Oberfläche, die als Kugelsegmente ausgebildet sind, die am Rand der Linse konvergieren; und
Bewirken einer Ionen-Implantation auf mindestens einer der Oberflächen der Linse, derart, daß die Oberfläche mit unterschiedlichen Werten des optischen Refraktions-Indexes versehen wird, die eine Mehrzahl konzentrischer optischer Zonen darstellen, wobei jede dieser Zonen einen Bereich mit höherer Korrektur-Brennstärke und einen Bereich mit niedriger Korrektur-Brennstärke aufweist, die untereinander durch stetig fortschreitende Zwischenbereiche verbunden sind.

8

## Revendications

1. Lentille ophtalmique (50, 72, 80, 82, 86) destinée à être portée par l'oeil pour donner une puissance variable de correction de vision, la lentille comprenant au moins une zone annulaire, la puissance correctrice variant radialement vers l'extérieur dans la zone annulaire d'une première valeur de correction de vision et une seconde valeur de correction de vision puis à une troisième valeur de correction de vision comprise entre la première et la seconde valeur de correction de vision, une partie au moins de la variation de la puissance correctrice dans la zone annulaire étant continue et progressive.

2. Lentille ophtalmique (50, 72, 80, 82, 86) selon la revendication 1, dans laquelle la puissance correctrice varie radialement vers l'extérieur de la seconde valeur de correction de vision à la première valeur de correction de vision.

3. Lentille ophtalmique (50, 72, 80, 82, 86) selon la revendication 1 ou 2, dans laquelle une partie au moins de la variation de puissance correctrice dans la zone annulaire entre la première et la seconde valeur de correction de vision et au moins une partie de la variation de la puissance correctrice dans la zone annulaire entre la seconde et la troisième valeur de correction de vision sont continues et progressives.

4. Lentille ophtalmique (50, 72, 80, 82, 86) selon l'une quelconque des revendications 1 à 3, dans laquelle la puissance correctrice dans la zone annulaire varie de façon continue et progressive en direction radiale pratiquement en totalité sur ladite zone.

5. Lentille ophtalmique (50, 72, 80, 82, 86) selon l'une quelconque des revendications 1 à 4, dans laquelle la lentille possède une zone centrale qui assure une correction de vision et la zone annulaire entoure la zone centrale.

6. Lentille ophtalmique (50, 72, 80, 82, 86) selon la revendication 5, dans laquelle la puissance correctrice de la zone centrale comprend une valeur de correction de vision qui est comprise entre la première et la seconde valeur de correction de vision.

7. Lentille ophtalmique (50, 72, 80, 82, 86) selon l'une quelconque des revendications 1 à 6, dans laquelle la lentille possède une seconde zone annulaire entourant la première zone annulaire, la seconde zone annulaire a, dans l'ordre radial vers l'extérieur, une quatrième valeur de correction de vision, des valeurs progressives de correction de vision, une cinquième valeur de correction de vision et des valeurs progressives de correction de vision, la quatrième et la cinquième valeur de correction de vision étant différentes et les valeurs progressives de correction de vision de la seconde zone annulaire comprenant des valeurs de correction de vision comprises entre la quatrième et la cinquième valeur de correction de vision.

8. Lentille ophtalmique (50, 72, 80, 82, 86) selon la revendication 7, dans laquelle la première et la quatrième valeur de correction de vision sont inférieures à la seconde et à la cinquième valeur de correction de vision respectivement.

9. Lentille ophtalmique (50, 72, 80, 82, 86) selon l'une quelconque des revendications précédentes, dans laquelle la première valeur de correction de vision est une puissance de correction en vision lointaine et la seconde valeur de correction de vision est une puissance de correction de vision rapprochée.

10. Lentille ophtalmique (50, 72, 80, 82, 86) selon l'une quelconque des revendications précédentes, dans laquelle la lentille opthalmique est une lentille intraoculaire (86).

11. Lentille ophtalmique (50, 72, 80, 82, 86) selon l'une quelconque des revendications 1 à 9, dans laquelle la lentille ophtalmique (80, 82) est destinée à être fixée à la cornée.

12. Lentille ophtalmique (50, 72, 80, 82, 86) selon l'une quelconque des revendications 1 à 9, dans laquelle la lentille ophtalmique est une lentille de contact (50).

13. Lentille ophtalmique (50, 72, 80, 82, 86) selon l'une quelconque des revendications précédentes, dans laquelle la lentille (72) a un indice de réfraction variant progressivement, assurant la variation continue

9

et progressive de la puissance correctrice.

14. Procédé de formation d'une lentille ophtalmique (72) qui comprend :

la préparation d'une lentille dont des surfaces antérieure et postérieure ont la configuration de segments de sphère qui convergent à la périphérie de la lentille, et

l'implantation ionique à une surface au moins de la lentille de manière que cette surface ait des valeurs variables d'indice de réfraction optique, formant plusieurs zones optiques concentriques, chacune des zones ayant une partie correctrice de puissance relativement élevée et une partie correctrice de puissance relativement faible, raccordées par des parties intermédiaires variant progressivement.

FIG. 1  PRIOR ART

EP 0 318 561 B1

FIG. 2 PRIOR ART

FIG. 3 PRIOR ART

FIG. 4

FIG. 5

EP 0 318 561 B1

FIG. 6a

FIG. 6b

FIG. 7

FIG. 8a

FIG. 8b

FIG. 8c